# EUROPEAN PATENT APPLICATION

(11) **EP 4 286 525 A1**
(43) Date of publication of application: **06.12.2023**
(21) Application number: 22745640.7
(22) Date of filing: 17.01.2022
(51) Int. Cl.: C12Q 1/02, G01N 30/04, G01N 30/26

(54) **METHOD FOR ANALYZING METABOLITES OF MICROORGANISMS**

(30) Priority: 26.01.2021 JP 2021010181
(71) Applicant: Shimadzu Corporation, Nakagyo-ku, Kyoto-shi, Kyoto 604-8511 (JP); National University Corporation Kobe University, Kobe-shi, Hyogo 657-8501 (JP); AJINOMOTO CO., INC., Chuo-ku Tokyo 104-8315 (JP)
(72) Inventor: HORIE, Shinnosuke, Kyoto-shi, Kyoto 604-8511 (JP); FUJITO, Yuka, Kyoto-shi, Kyoto 604-8511 (JP); HASUNUMA, Tomohisa, Kobe-shi, Hyogo 657-8501 (JP); YOSHIDA, Takanobu, Kobe-shi, Hyogo 657-8501 (JP); URAHATA, Erika, Kawasaki-shi, Kanagawa 210-8681 (JP)
(74) Representative: Müller-Boré & Partner Patentanwälte PartG mbB
(86) International application number: PCT/JP2022/001459
(87) International publication number: WO 2022/163417

(57) **Abstract**

Provided is a method capable of shortening the time required for analysis and capable of analyzing various substances produced by microorganisms. A method for analyzing a metabolite of a microorganism according to the present invention includes: a step of supplying a mobile phase including carbon dioxide in a liquid state, a subcritical state, or a supercritical state to a container in which a microorganism cultured in a medium is contained together with the medium, to move a component of a metabolite of the microorganism present in the microorganism and the medium to the mobile phase; a step of introducing a mobile phase to which a component of the metabolite has moved into a column; and a step of performing mass spectrometry on a component of the metabolite contained in the mobile phase that has passed through the column.

## Description

### TECHNICAL FIELD

The present invention relates to a method for analyzing a metabolite of a microorganism.

### BACKGROUND ART

In recent years, it has been attempted to obtain a useful substance from a metabolite of a microorganism (recombinant. hereinafter referred to as "recombinant microorganism") by subjecting the microorganism to genetic recombination operation to make the microorganism acquire the ability to produce the useful substance, or to enhance the production ability. For example, Patent Literature 1 recites that isoprene or terpene is obtained from bacteria belonging to the genus Clostridium or bacteria belonging to the genus Moorella subjected to genetic recombination operations. Isoprene is a hydrocarbon represented by the structural formula CH₂=C(CH₃)CH=CH₂. Terpene is a generic term for various substances having different molecular structures and including isoprenes as constituent units. These isoprene and terpene are used for various uses such as raw materials of resins, raw materials of fragrances, food additives, detergents, electronic materials, and pharmaceutical and agrochemical raw materials.

One of the methods for investigating whether a microorganism has acquired an expected ability by genetic recombination operation is to analyze a metabolite of the microorganism using a chromatograph mass spectrometer. Patent Literature 1 describes that a recombinant microorganism (bacterial strain) grown by culturing on an agar medium is inoculated into a liquid medium, this liquid medium is charged into a vial container together with a mixed gas of carbon monoxide, carbon dioxide, and hydrogen, and the mixture is hermetically sealed and cultured, and then a gas phase portion (gas in an upper space (head space) in the vial container) is analyzed by a gas chromatograph mass spectrometer. Since isoprene contained in the metabolite of the recombinant microorganism is volatilized from the liquid medium through culture in a vial container, the production ability of isoprene of the recombinant microorganism can be investigated by analyzing the gas phase portion.

### CITATION LIST

### PATENT LITERATURE

Patent Literature 1: WO 2018/155272 A
Patent Literature 2: JP 2019-184502 A
Patent Literature 3: WO 2016/084963 A

### SUMMARY OF INVENTION

### TECHNICAL PROBLEM

In the method described in Patent Literature 1, it is necessary to culture a recombinant microorganism in a liquid medium to sufficiently volatilize a desired substance (isoprene) contained in a metabolite of the recombinant microorganism, and it takes time to analyze the substance. In addition, when a substance produced by a microorganism does not have volatility, the substance cannot be analyzed.

An object of the present invention is to provide a method capable of shortening the time required for analysis and capable of analyzing various substances produced by microorganisms.

### SOLUTION TO PROBLEM

A method for analyzing a metabolite of a microorganism according to the present invention, which has been made to solve the above problems, includes:
a step of supplying a mobile phase including carbon dioxide in a liquid state, in a subcritical state, or in a supercritical state to a container in which a microorganism cultured in a medium is contained together with the medium, to move a component of a metabolite of the microorganism contained in the microorganism and the medium to the mobile phase;
a step of introducing the mobile phase to which the component of the metabolite has moved to a column; and
a step of performing mass spectrometry on the component of the metabolite contained in the mobile phase that has passed through the column.

### ADVANTAGEOUS EFFECTS OF INVENTION

In the method according to the present invention, in a state where a microorganism cultured in a medium is contained in a container together with the medium, carbon dioxide is fed at a high pressure (7.38 MPa or more), and carbon dioxide in a liquid state, in a subcritical state, or in a supercritical state is supplied to the container. When the mobile phase including carbon dioxide that has been brought into a liquid state, in a subcritical state, or in a supercritical state under high pressure comes into contact with the microorganism and the culture medium in the container, the components of the substance to be analyzed (metabolite) contained in the microorganisms and the culture medium move to the mobile phase. The mobile phase to which the metabolite components have moved in this manner is introduced into a column, and the components of substances contained in the mobile phase that has passed through the column are subjected to mass spectrometry.

With the method according to the present invention, the component of the substance to be analyzed produced by the microorganism is moved to the mobile phase including carbon dioxide in the liquid state, in the subcritical state, or in the supercritical state, and therefore it is not necessary to culture the recombinant microorganism in the liquid medium in order to volatilize the component of the substance to be analyzed, whereby the time required for analysis can be shortened. In addition, the component of the substance to be analyzed does not need to have volatility, and therefore various substances can be analyzed.

### BRIEF DESCRIPTION OF DRAWINGS

Fig. 1 is a schematic configuration diagram showing an example of a supercritical fluid extraction-supercritical fluid chromatograph mass spectrometer (SFE-SFC-MS) used in performing an embodiment of a method for analyzing a metabolite of a microorganism according to the present invention.
Fig. 2 is a flowchart showing a method for analyzing a metabolite of a microorganism according to the present embodiment.
Figs. 3A to 3G are diagrams showing a procedure of cutting out a colony of a microorganism cultured on an agar medium together with a part of the agar medium and containing them in a container in the method of the present embodiment.
Fig. 4 is a graph showing an example of a time course in a mixing ratio of a modifier in a mobile phase used in the method of the present embodiment.
Figs. 5A to 5C are chromatograms obtained by moving a component of a metabolite from a terpene-non-producing strain (Fig. 5A), a terpene-producing strain (Fig. 5B), and a terpene-high-producing strain (Fig. 5C) to a mobile phase by using the method of the present embodiment.
Fig. 6 is a graph showing peak areas of chromatogram obtained using the method of the present embodiment and a conventional method for a terpene-non-producing strain, a terpene-producing strain, and a terpene-high-producing strain.

### DESCRIPTION OF EMBODIMENTS

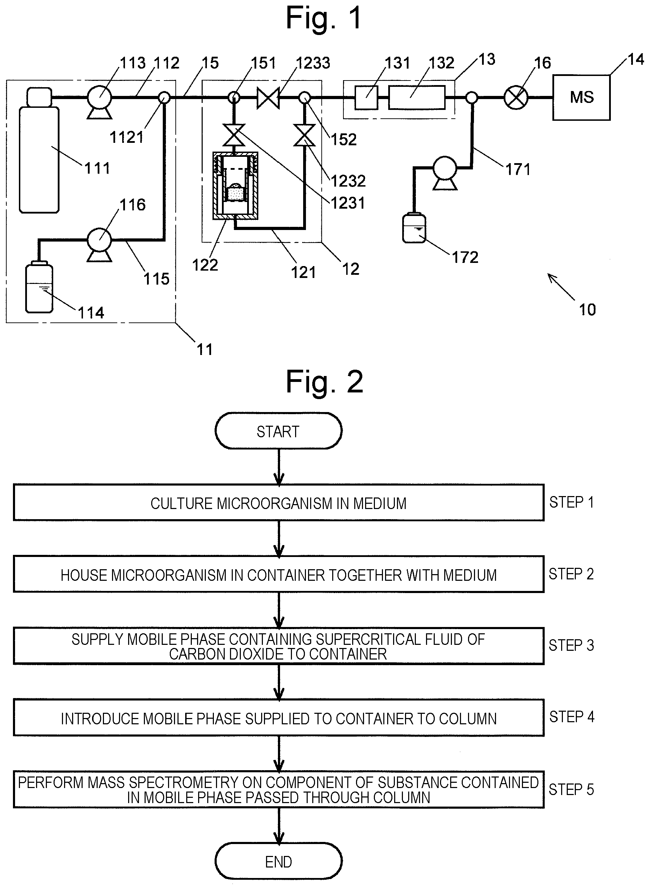

An embodiment of a method for analyzing a metabolite of a microorganism according to the present invention will be described with reference to Figs. 1 to 6.

### (1) Apparatus for performing method for analyzing metabolite of microorganism according to present embodiment

Fig. 1 schematically illustrates a configuration of a chromatograph mass spectrometer used when performing the method for analyzing a metabolite of a microorganism of the present embodiment. Note that this chromatograph mass spectrometer is called a supercritical fluid extraction-supercritical fluid chromatograph mass spectrometer (refer to Patent Literature 2) since it not only feeds carbon dioxide but also has a pressurizing function for bringing the carbon dioxide into a supercritical state, and is hereinafter referred to as "SFE-SFC-MS". Note that "SFE" is an abbreviation for "Supercritical Fluid Extraction", "SFC" is an abbreviation for "Supercritical Fluid Chromatograph", and "MS" is an abbreviation for "Mass Spectrometer". However, as described later, in the present embodiment, carbon dioxide in not only the supercritical state but also the subcritical state and the liquid state is used as an extraction liquid or a mobile phase.

An SFE-SFC-MS 10 includes a liquefied carbonic acid feeding unit 11, a supercritical fluid extraction (SFE) unit 12, a supercritical fluid chromatograph (SFC) unit 13, and a mass spectrometry (MS) unit 14.

The liquefied carbonic acid feeding unit 11 includes a gas cylinder 111, a gas flow path 112, a pressurizing pump 113, a modifier container 114, a modifier flow path 115, and a liquid feeding pump 116.

The gas cylinder 111 is a carbon dioxide cylinder, and supplies carbon dioxide to the gas flow path 112. The pressurizing pump 113 is provided in the gas flow path 112, and maintains carbon dioxide in a liquid state, a subcritical state, or a supercritical state by pressurizing the carbon dioxide to a pressure of 7.38 MPa or more.

The modifier container 114 is a container that contains a modifier. A modifier is also called an entrainer. A modifier is an auxiliary solvent mainly having polar molecules, and is one added to carbon dioxide in order to facilitate movement of components contained in metabolites. The modifier to be used can be appropriately changed by replacing the modifier container 114 according to the metabolite to be analyzed. The modifier flow path 115 is one connecting the modifier container 114 and the gas flow path 112, and joins the gas flow path 112 at a first junction 1121. The liquid feeding pump 116 is provided in the modifier flow path 115. The modifier is introduced from the modifier container 114 into the gas flow path 112 through the modifier flow path 115 by the action of the liquid feeding pump 116, and is mixed with carbon dioxide flowing through the gas flow path 112. Hereinafter, the flow path downstream of the first junction 1121 as viewed from the gas flow path 112 is referred to as mobile phase flow path 15.

An SFE unit 12 is provided in the mobile phase flow path 15, and includes a branch flow path 121, a sample container 122 (corresponding to "container" in the present invention), and first to third on-off valves 1231 to 1233. The branch flow path 121 branches off from the mobile phase flow path 15 at a branch point 151 in the mobile phase flow path 15, and joins the mobile phase flow path 15 at a second junction 152 on the downstream side of the branch point 151 in the mobile phase flow path 15. The sample container 122 is a container that is provided in the branch flow path 121 and contains a sample 20 in which colonies 22 of microorganisms and a part of an agar medium 21 around the colonies are combined as described later. The first on-off valve 1231 is an on-off valve provided in the branch flow path 121 between the branch point 151 and the sample container 122, the second on-off valve 1232 is an on-off valve provided in the branch flow path 121 between the sample container 122 and the second junction 152, and the third on-off valve 1233 is an on-off valve provided in the mobile phase flow path 15 between the branch point 151 and the second junction 152.

The SFC unit 13 is provided on the downstream side of the SFE unit 12 of the mobile phase flow path 15, and includes a trap column 131 and an SFC analysis column 132 disposed in series in order from the upstream side. The trap column 131 and the SFC analysis column 132 are filled with different fillers. As the filler filled in the trap column 131, a filler that more easily traps the substance to be analyzed than the filler filled in the SFC analysis column 132 is used. Note that the SFC analysis column 132 may serve as the trap column 131 without using the trap column 131.

In the present embodiment, an MS/MS type mass spectrometer is used as the MS unit 14, but other mass spectrometers may be used.

A back pressure control valve 16 is provided in the mobile phase flow path 15 between the SFC unit 13 and the MS unit 14. The back pressure control valve 16 is provided to maintain the pressure of the mobile phase so that the state of the mobile phase can be maintained on the upstream of the back pressure control valve 16.

A makeup flow path 172 is provided between the SFC unit 13 and the back pressure control valve 16 in the mobile phase flow path 15, and a makeup container 171 that stores a makeup solution is connected to the makeup flow path 172. The makeup solution is a liquid for promoting the detection of the component to be analyzed by the MS unit 14, and methanol, ethanol, or the like can be used, and a salt such as ammonium formate can be added to methanol or the like as necessary.

### (2) Method for analyzing metabolite of microorganism according to the present embodiment

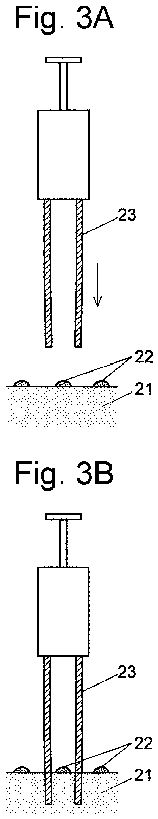

Hereinafter, the details of the method for analyzing a metabolite of a microorganism of the present embodiment will be described with reference to Figs. 2 and 3A to 3G. Microorganisms are previously cultured on the agar medium 21 by a typical method to form colonies 22 of the microorganisms (step 1 in Fig. 2, refer to Fig. 3A).

Herein, the metabolite of microorganisms refers to an organic compound that is accumulated in cells in a culture process or discharged into a culture medium, and also includes a fermentation product. Examples of the organic compound that is a metabolite of microorganisms include a hydrocarbon, an amino acid, an organic acid, polysaccharides, a protein, an antibiotic, and an alcohol.

Examples of the hydrocarbon include an aliphatic hydrocarbon, an aromatic hydrocarbon, and an alicyclic hydrocarbon, and examples of the aliphatic hydrocarbon include: alkanes such as ethyl, propane, butane, pentane, hexane, heptane, octane, nonane, decane, undecane, dodecane, heptadecane, and octadecane; alkenes such as ethylene, propylene, butene, pentene, hexene, octene, nonene, decene, butadiene, and isoprene; and alkynes such as ethyne, propyne, butyne, and pentyne. Examples of the aromatic hydrocarbon include benzene, toluene, xylene, and ethylbenzene. Examples of the alicyclic hydrocarbon include cyclopropane, cyclobutane, cyclopentane, cyclohexane, cycloheptane, cyclooctane, cyclobutene, cyclopentene, cyclohexene, cycloheptene, and cyclooctene.

Of the aliphatic hydrocarbon, the aromatic hydrocarbon, and the alicyclic hydrocarbon, a substance insoluble in water is also referred to as oil-soluble component. Specific examples of the oil-soluble component include an isoprenoid. The isoprenoid is a hydrocarbon having isoprene as a constituent unit, and is a substance biosynthesized by an isoprenoid pathway. Examples of the isoprenoid include a terpene, a terpenoid, a steroid, and a carotenoid. Examples of the terpene include monoterpene, sesquiterpene, diterpene, sesterterpene, triterpene, and tetraterpene. Examples of the terpenoid include a monoterpenoid, a sesquiterpenoid, a diterpenoid, a sestaterpenoid, a triterpenoid, and a tetraterpenoid. The steroid is a generic term for compounds having a cyclopentanohydrophenanthrene ring skeleton, and examples of the steroid include β-estradiol and progesterone. The carotenoid refers to a compound having a basic skeleton of C₄₀H₅₆ made of 8 isoprene units, and examples of the carotenoid include carotene, lycopene, lutein, zeaxanthin, and astaxanthin.

Examples of the amino acid include glycine, alanine, valine, leucine, isoleucine, serine, threonine, cysteine, cystine, methionine, phenylalanine, tyrosine, tryptophan, proline, hydroxyproline, asparagine, glutamine, aspartic acid, glutamic acid, lysine, histidine, and arginine. Individual amino acids can be classified into one of acidic amino acids, basic amino acids, and neutral amino acids depending on the differences in side chains. Specifically, glutamic acid and aspartic acid can be classified into acidic amino acids, lysine, arginine and histidine can be classified into basic amino acids, and glycine, alanine, threonine, methionine, cysteine, serine, valine, leucine, isoleucine, tryptophan, phenylalanine, tyrosine, proline, glutamine, and asparagine can be classified into neutral amino acids.

Examples of the organic acid include acetic acid, lactic acid, pyruvic acid, succinic acid, malic acid, itaconic acid, citric acid, acrylic acid, propionic acid, and fumaric acid.

Examples of the polysaccharides include xanthan, dextran, alginate, hyaluronic acid, curdlan, geran, sucrose glucan, and pullulan. Examples of the protein include a hormone, a lymphokine, an interferon, and an enzyme (amylase, glucoamylase, invertase lactase, protease, lipase, and the like).

Examples of the antibiotic include a fungicide (a β-lactam, a macrolide, an ansamycin, a tetracycline, a chloramenicol, a peptide antibiotic, an aminoglycoside, and the like), an antifungal agent (polyoxine B, griseofulvin, polyene macrolide, and the like), an anticancer agent (daunomycin, adriamycin, dactinomycin, misramycin, bleomycin, and the like), a protease/peptidase inhibitor (leupeptin, antipain, pepstatin, and the like), and a cholesterol biosynthesis inhibitor (compactin, lovastatin, pravastatin, and the like).

Examples of the alcohol include ethanol, isopropanol, glycerin, propylene glycol, trimethylene glycol, 1-butanol, and sorbitol. In addition, examples of the organic compound produced by the fermentation method include organic compounds such as acrylamide, diene compounds (isoprene and the like), and pentanediamine.

A technique for producing an organic compound as described above by culturing a microorganism capable of producing an organic compound is widely known. The present embodiment can be widely applied to such a technique. In microbial fermentation, microorganisms also grow themselves by using a carbon source, a nitrogen source, or the like. In this sense, in the present embodiment, microbial cells are also included in the metabolite. Examples of the microbial cell include any microorganism capable of producing an organic compound.

Microorganisms capable of producing an organic compound include both microorganisms inherently capable of producing an organic compound and microorganisms inherently incapable of producing an organic compound or substantially incapable of producing an organic compound but having acquired the ability to produce an organic compound after introduction of an organic compound-producing gene by genetic recombination. Regarding microorganisms capable of producing an organic compound, various microorganisms are known depending on the type of organic compound, and in the present embodiment, these known microorganisms can be widely used.

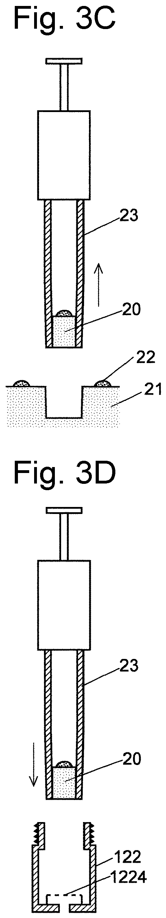

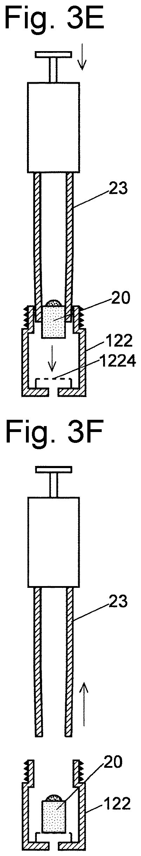

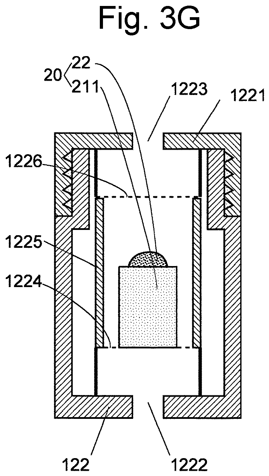

Then, by the method described below, the colonies 22 of microorganisms cultured on the agar medium 21 are contained in the sample container 122 together with a part of the agar medium 21 (referred to as "agar medium piece 211") (step 2). First, the tip of a pipette 23 is pierced into a partial area of the agar medium 21 including the position where the colonies 22 are formed (Figs. 3A and 3B), and the pipette 23 is pulled out from the agar medium 21 (Fig. 3C). As a result, the sample 20 including the colonies 22 and the agar medium piece 211 is cut in the pipette 23. Then, the tip of the pipette 23 is inserted into the sample container 122 (Fig. 3D), and the sample 20 in the pipette 23 is pushed from the pipette 23 into the sample container 122 having a first filter 1224 described later placed on the bottom portion (Fig. 3E). Thereafter, the tip of the pipette 23 is taken out from the sample container 122 (Fig. 3F), a cylindrical body 1225 and a second filter 1226 described later are contained in the sample container 122, and a lid 1221 is attached to the sample container 122 (Fig. 3G).

A bottom opening 1222 is provided in the bottom portion of the sample container 122, and a lid opening 1223 is provided in the lid 1221. The first filter 1224 is provided to cover the bottom opening 1222, and the cylindrical body 1225 is placed on the first filter 1224. The sample 20 is contained inside the cylindrical body 1225. The second filter 1226 is placed on the cylindrical body 1225. With the above configuration, the flow path of the mobile phase from the lid opening 1223 toward (or away from) the bottom opening 1222 through the second filter 1226, the cylindrical body 1225 (the sample 20 in the cylindrical body 1225), and the first filter 1224 is formed in the sample container 122.

Then, the sample container 122 is attached to the SFE unit 12 of the SFE-SFC-MS 10, and the bottom opening 1222 and the lid opening 1223 of the sample container 122 are connected to the branch flow path 121, so that the inside of the sample container 122 and the branch flow path 121 communicate with each other by a method similar to that used in the conventional SFE-SFC-MS 10.

Then, in a state where the first on-off valve 1231 is closed and the third on-off valve 1233 is opened, carbon dioxide gas is supplied from the gas cylinder 111 to the gas flow path 112, and a predetermined pressure is applied to the carbon dioxide gas by the pressurizing pump 113. In addition, as necessary, the modifier is supplied from the modifier container 114 to the modifier flow path 115 by the liquid feeding pump 116, and the modifier is mixed with carbon dioxide at the first junction 1121. As a result, a mobile phase including carbon dioxide in a liquid state, a subcritical state, or a supercritical state is obtained. Note that, as described later, the supply of the modifier need not be performed in a part of the analysis time, or in a case where the substance (metabolite) to be analyzed includes nonpolar molecules, the supply of the modifier need not be performed over the entire analysis time.

Then, the component of the metabolite that is metabolized from the microorganism and is present in the colonies 22 and the agar medium piece 211 is moved to the mobile phase by supplying the mobile phase to the sample container 122 by the method described below (Step 3: SFE). Herein, the SFE method includes two methods of static extraction and dynamic extraction.

In the static extraction, first, the mobile phase is supplied into the sample container 122 by opening the second on-off valve 1232 in a state where the first on-off valve 1231 is closed and the third on-off valve 1233 is opened. Then, the second on-off valve 1232 is closed when a predetermined amount of the mobile phase is supplied into the sample container 122. As a result, the component of the metabolite moves to the mobile phase stored in the sample container 122 (without flowing the mobile phase). Further, after a lapse of a predetermined time, the first on-off valve 1231 and the second on-off valve 1232 are opened to close the third on-off valve 1233. As a result, the component moved to the mobile phase is discharged from the sample container 122 together with the mobile phase. Note that, in the step of supplying the mobile phase into the sample container 122, instead of opening the second on-off valve 1232 to close the first on-off valve 1231, the first on-off valve 1231 may be opened to close the second on-off valve 1232.

In the dynamic extraction, the first on-off valve 1231 and the second on-off valve 1232 are opened, and the third on-off valve 1233 is closed. As a result, the component of the metabolite moves into the mobile phase flowing so as to pass through the sample container 122, and the component is discharged from the sample container 122 together with the mobile phase.

In the present embodiment, only one of the static extraction and the dynamic extraction may be performed, or both may be performed.

The component of the metabolite moved to the mobile phase is introduced into the trap column 131 of the SFC unit 13 together with the mobile phase, and is captured by the trap column 131.

After the step 3 (SFE) is completed by the above operation, the first on-off valve 1231 and the second on-off valve 1232 are closed, and then the third on-off valve 1233 is opened to supply the mobile phase (not containing the metabolite component) into the trap column 131. As a result, the component of the metabolite trapped in the trap column 131 is discharged from the trap column 131, introduced into the SFC analysis column 132, and temporally separated for each component in the SFC analysis column 132 (step 4: SFC).

In this case, by adjusting the mixing ratio of the modifier contained in the mobile phase, it is possible to control the discharge rate of the component of the metabolite from the trap column 131 and the retention time of the component in the SFC analysis column 132. For example, by increasing the mixing ratio of the modifier, the discharge of the metabolite component from the trap column 131 is promoted. In addition, the mixing ratio of the modifier is increased at the start of discharge from the trap column 131, and then the mixing ratio is decreased, whereby while the discharge from the trap column 131 is promoted, the retention time in the SFC analysis column 132 is increased, and the accuracy of time separation for each component can be increased. Specific examples of the type and mixing ratio of the modifier will be described later.

Each component time-separated by the SFC analysis column 132 is introduced into the MS unit 14 and subjected to mass spectrometry (step 5). This makes it possible to quantify a target useful substance contained in the metabolite.

With the method of the present embodiment, since the component of the substance to be analyzed produced by the microorganism is moved to the mobile phase including carbon dioxide, it is not necessary to culture the recombinant microorganism in a liquid medium in order to volatilize the component of the substance to be analyzed. Therefore, the time required for analysis can be shortened. In addition, the component of the substance to be analyzed does not need to have volatility, and therefore various substances can be analyzed.

In the present embodiment, the colonies 22 of the microorganism generated on the agar medium 21 are cut out together with a part of the agar medium (the agar medium piece 211) and contained in the container as it is. Besides this method, a method of scraping the colonies 22 from the agar medium 21 and containing the colonies in the sample container 122 can also be adopted, but with this method, it takes time and effort to scrape the colonies, and it is difficult to reliably scrape the entire colonies of the microorganism generated on the agar medium. By contrast, with the method of the present embodiment, the entire colonies 22 can be easily collected in a short time. Note that, in the present invention, the method for containing the microorganism in a container together with the culture medium is not limited to the method used in the present embodiment.

### (3) Specific examples of quantitative analysis on metabolite

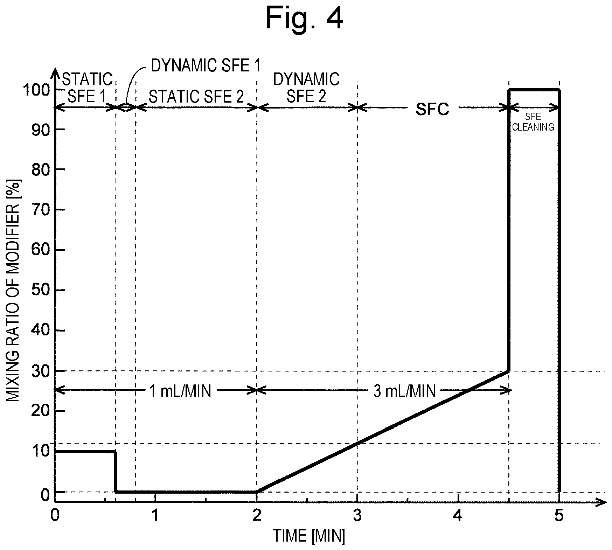

Hereinafter, the results of an experiment of quantitative analysis on a metabolite will be described with reference to Figs. 4 to 6. In this experiment, (a) a wild E. coli strain having no ability to produce a terpene (Escherichia coli (hereinafter, referred to as "E. coli") MG 1655 strain, accession number NITE BP-1686, hereinafter referred to as "terpene-non-producing strain"), (b) a strain in which the ability to produce a terpene was imparted by performing a genetic recombination operation on a terpene-non-producing strain (terpene-producing strain), and (c) a strain in which the ability to produce a terpene was imparted higher than that of the terpene-producing strain by performing a genetic recombination operation different from that in (b) on a terpene-non-producing strain (terpene-high-producing strain) were used as microorganisms, and limonene (C₁₀H₁₆), which is a monoterpene contained in the metabolite of these three types of microorganisms, was used as an object to be analyzed.

Terpene (isoprene) is a hydrocarbon used as a material of isoprene-based rubber, and limonene, which is a type of terpene, is an oil-soluble compound known as one of aroma components contained in citrus fruits.

(b) The terpene-producing strain was obtained by preparing competent cells of (a) a terpene-non-producing strain, and introducing a plasmid pACYC177-Ptac-opt_LMS-ispA* expressing a limonene synthase gene and a farnesyl diphosphate synthase gene by electroporation. In addition, (c) the terpene-high-producing strain was obtained by constructing an MG1655 poxB::Ptac-dxs strain in which the dxs gene was enhanced at the poxB locus on the chromosome of a terpene-non-producing strain, preparing a competent cell of this strain, and introducing a plasmid pACYC177-Ptac-opt_LMS-ispA* expressing a limonene synthase gene and a farnesyl diphosphate synthase gene by electroporation.

The plasmid used for constructing (b) the terpene-producing strain and (c) the terpene-high-producing strain was constructed as follows according to the procedure described in Patent Literature 3. Note that the following procedure is described in Patent Literature 3, and thus detailed description of the procedure is omitted herein.

### (1) Obtainment of plant-derived limonene synthase gene

In order to efficiently express a plant-derived limonene synthase gene, the secondary structure was eliminated, the codon was optimized so as to be identical to the codon usage of P. ananatis, and the chloroplast localization signal was further cleaved. The gene thus obtained was named opt_LMS. Note that examples of the plant of plant-derived limonene synthase include Picea sitchensis, Abies grandis, Mentha spicata, Citrus unshiu, and Citrus limon. In this example, limonene synthase derived from spearmint was used.

### (2) Obtainment of mutant farnesyl diphosphate synthase gene derived from E. coli

In order to efficiently express the ispA mutant (S80F) gene having a high geranyl diphosphate producing activity, which is a mutant strain of the ispA gene encoding farnesyl diphosphate synthase of E. coli, there was obtained a mutant farnesyl diphosphate synthase gene derived from E. coli in which codons were optimized to eliminate secondary structures. This gene was named ispA*.

### (3) Construction of plasmid for co-expression of opt_LMS and ispA* genes

Genes of opt_LMS and ispA* were chemically synthesized, and then cloned into pUC57 (manufactured by GenScript) as a standard vector to obtain plasmids. The resulting plasmids were named pUC57-opt_LMS and pUC57-ispA*, respectively.

Then, PCR was performed using a predetermined primer and pUC57-opt_LMS as a template, and PCR was further performed using the obtained PCR fragment to obtain a pUC57-opt_LMS fragment. In addition, PCR was performed using pUC57-ispA* as a template using a predetermined primer to obtain a pUC57-ispA* fragment. The pUC57-opt_LMS fragment and the pUC57-ispA* fragment were ligated to pACYC177 (manufactured by NIPPON GENE CO., LTD.) digested with restriction enzymes Pstl and Pcal using In-Fusion HD cloning kit (manufactured by Clontech Laboratories, Inc.) to obtain a plasmid for co-expression of opt_LMS and ispA* genes. This was named pACYC177-Ptac-opt_ LMS-ispA*.

These three strains were analyzed by a conventional quantitative analysis method. Specifically, the above three kinds of bacterial strains were cultured on an agar medium for growth, then bacterial cells on the surface of the medium were scraped off, and inoculated in a liquid medium for limonene fermentation contained in a closed container, and further cultured. After completion of the culture, the gas components in the closed container were measured by gas chromatography using a quantitative analyzer. As a result, limonene was not detected in the terpene-non-producing strain, and limonene was detected in the terpene-producing strain and the terpene-high-producing strain. The detection amount of limonene in the terpene-high-producing strain was about 5 times the detection amount in the terpene-producing strain.

Detailed conditions in the analysis using the method of the present embodiment will be described.

Samples were prepared as follows. Each bacterial strain was cultured on the agar medium 21 in the presence of oxygen as a growth promoter until the diameter of the colonies 22 reached about 3 mm, and then the supply amount of oxygen was reduced and the bacterial strain was further cultured. Then, the colonies 22 and the agar medium 21 in the range of 5 mm in diameter around the colonies were cut out by the above-described method, and the colonies 22 and the agar medium pieces 211 were contained in the container.

In SFE (step 3), ammonium formate methanol having an ammonium formate concentration of 15 mmol/L (millimole per liter) was used as a modifier, and extraction of metabolites was performed by combining static extraction and dynamic extraction as follows (refer to Fig. 4). First, a mobile phase having a mixing ratio (volume ratio) of the modifier of 10% was supplied to the sample container 122 at a pressure of 15 MPa and a supply rate of 1 mL/min for 0.6 minutes without flowing out of the sample container 122, and static extraction ("static SFE 1" in Fig. 4) was performed. Then, while allowing the mobile phase in the sample container 122 to flow out, the mobile phase not containing the modifier (with a mixing ratio of 0%) was supplied to the sample container 122 at a pressure of 15 MPa and a supply rate of 1 mL/min for 0.2 minutes, and dynamic extraction ("dynamic SFE 1" in Fig. 4) was performed. As a result, the metabolite moved to the mobile phase in the sample container 122 was discharged to the SFC unit 13 by the mobile phase supplied to the sample container 122. Subsequently, the mobile phase not containing the modifier was supplied to the sample container 122 at a pressure of 15 MPa and a supply rate of 1 mL/min for 1.2 minutes without causing the mobile phase to flow out of the sample container 122, and static extraction ("static SFE 2" in Fig. 4) was performed. Immediately thereafter, while allowing the mobile phase to flow out of the sample container 122, the mobile phase was supplied to the sample container 122 at a pressure of 15 MPa and a supply rate of 3 mL/min while continuously increasing the mixing ratio of the modifier in the mobile phase from 0% to 12% in 1 minute, and dynamic extraction ("dynamic SFE 2" in Fig. 4) was performed.

In SFC (step 4), the mobile phase was supplied to the SFC unit 13 at a pressure of 15 MPa and a supply rate of 3 mL/min while continuously increasing the mixing ratio of the modifier in the mobile phase from 12% to 30% in 1.5 minutes (Fig. 4). The temperature of the SFC analysis column 132 was maintained at 40 °C.

The eluate eluted from the SFC analysis column 132 was mixed with the makeup solution containing methanol supplied from the makeup container 171 at a supply rate of 0.1 mL/min and introduced into the MS unit 14.

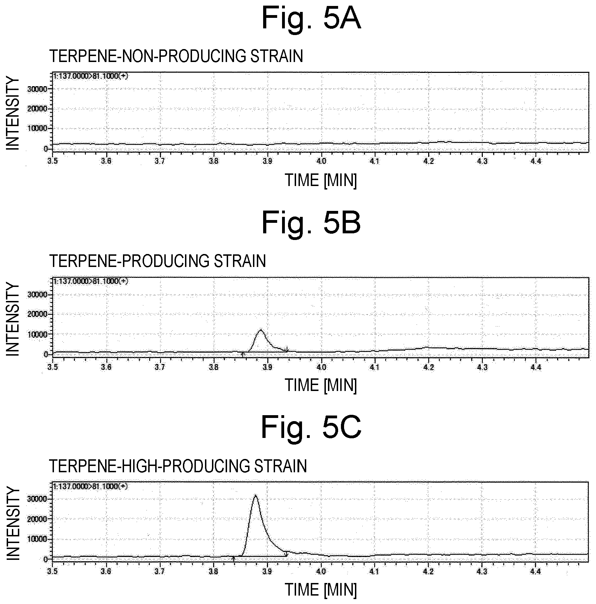

An example of the obtained chromatogram is shown in Figs. 5A to 5C for each of the terpene-non-producing strain (Fig. 5A), the terpene-producing strain (Fig. 5B), and the terpene-high-producing strain (Fig. 5C). At the retention time at which no peak appears in the chromatogram of the terpene-non-producing strain, a peak derived from limonene appears in the chromatograms of the terpene-producing strain and the terpene-high-producing strain. The peak area is larger in the terpene-high-producing strain than in the terpene-producing strain.

When the inside of the sample container 122 was checked after the end of the analysis, the agar medium pieces 211 remained in substantially the same shape and size as before the analysis. Therefore, it is considered that the agar medium pieces 211 hardly flow out at the time of extracting the metabolites and do not adversely affect the analysis.

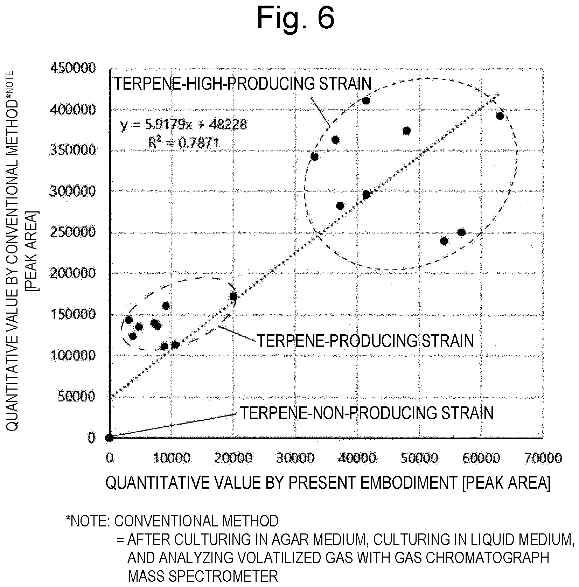

Then, for each of the terpene-producing strain and the terpene-high-producing strain, chromatograms were acquired with a plurality of samples to obtain peak areas, and a part of each of the plurality of samples was collected to obtain peak areas of the chromatograms acquired by the conventional method (culturing in liquid medium to volatilize components of metabolites and analyzing with gas chromatograph). Fig. 6 shows a graph in which the peak area obtained by the method of the present embodiment is taken on the horizontal axis and the peak area obtained by the conventional method for the same sample is taken on the vertical axis. From this graph, it can be found that as the peak area obtained by the conventional method is larger, the peak area obtained by the method of the present embodiment is also larger, and there is a correlation between the peak area obtained by the conventional method and the peak area obtained by the method of the present embodiment. That is, the same results as those of the conventional method, which is an already established analysis method, are obtained, and therefore it can be said that the analysis results obtained by the method of the present embodiment are valid.

### (4) Modifications

In the above embodiment, the colonies 22 of microorganisms are collected together with a part 211 of the agar medium 21 using the pipette 23, but the microorganisms collected by other methods may be contained in the sample container 122 together with the medium. Instead of culturing the microorganisms outside the sample container 122, the microorganisms may be cultured in a culture medium contained in the sample container 122. In other respects, the present invention is not limited to the above embodiment, and various modifications can be made in accordance with the gist of the invention.

### [Modes]

It will be understood by those skilled in the art that the exemplary embodiments described above are specific examples of the following modes.

### (Clause 1)

A method for analyzing a metabolite of a microorganism according to clause 1 includes:
a step of supplying a mobile phase including carbon dioxide in a liquid state, in a subcritical state, or in a supercritical state to a container in which a microorganism cultured in a medium is contained together with the medium, to move a component of a metabolite of the microorganism contained in the microorganism and the medium to the mobile phase;
a step of introducing the mobile phase to which the component of the metabolite has moved to a column; and
a step of performing mass spectrometry on the component of the metabolite contained in the mobile phase that has passed through the column.

In the method for analyzing a metabolite of a microorganism according to clause 1, by extracting a metabolite of the microorganism using a solvent containing carbon dioxide in a liquid state, in a subcritical state, or in a supercritical state in a state where the microorganism cultured on the culture medium is contained in a container together with the culture medium, a large amount of the metabolite can be extracted in a short time as compared with a case where the metabolite is extracted in the form of a volatile. Therefore, the metabolite of the microorganism can be quantified with high accuracy in a short time.

The microorganism may be contained in the container together with the culture medium after being cultured outside the container, or may be cultured in the container from the beginning.

The mobile phase may be carbon dioxide alone or a mobile phase obtained by mixing carbon dioxide and a modifier. Herein, the modifier is one added to carbon dioxide in order to facilitate extraction of a substance and to elute the substance from the column in a predetermined time. The modifier can be appropriately selected from materials of a mobile phase in a normal liquid chromatograph according to a metabolite to be analyzed and a type of a column to be used.

### (Clause 2)

A method for quantifying a metabolite of a microorganism according to clause 2 includes, in the method according to clause 1:
culturing the microorganism on an agar medium outside the container;
cutting the microorganism together with a part of the agar medium; and
containing the microorganism in the container together with a part of the agar medium.

When the microorganism is cultured on the agar medium outside the container, a method of scraping the microorganism from the agar medium can be adopted, besides the method of collecting the microorganism used in the method of quantifying the metabolite of the microorganism according to clause 2. However, with this method, it takes time and effort to scrape, and it is difficult to reliably scrape the entire colonies of the microorganism generated on the agar medium. In contrast, with the method for quantifying a metabolite of a microorganism according to clause 2, the microorganism generated on the agar medium is cut out together with a part of the agar medium and contained in the container as it is, and thus the entire microorganism (colonies of the microorganism) can be easily collected in a short time.

### (Clause 3)

In a method for quantifying a metabolite of a microorganism according to clause 3, in the method according to clause 1 or 2,
a component of a metabolite of the microorganism is an oil-soluble component, and
the mobile phase is a mixture of carbon dioxide and ammonium formate methanol.

With the method for quantifying a metabolite of a microorganism according to clause 3, it is possible to efficiently move an oil-soluble component such as terpene to a mobile phase by using a mobile phase that is a mixture of carbon dioxide and ammonium formate methanol.

### REFERENCE SIGNS LIST

10... Supercritical Fluid Extraction-Supercritical Fluid Chromatograph Mass Spectrometer (SFE-SFC-MS)
11... Liquefied Carbonic Acid Feeding Unit
111... Gas Cylinder
112... Gas Flow Path
1121... First Junction
113... Pressurizing Pump
114... Modifier Container
115... Modifier Flow Path
116... Liquid Feeding Pump
12... Supercritical Fluid Extraction (SFE) Unit
121... Branch Flow Path
122... Sample Container
1221... Lid
1222... Bottom Opening
1223... Lid Opening
1224... First Filter
1225... Cylindrical Body
1226... Second Filter
1231... First On-Off Valve
1232... Second On-Off Valve
1233... Third On-Off Valve
13... Supercritical Fluid Chromatograph (SFC) Unit
131... Trap Column
132... SFC Analysis Column
14... Mass Spectrometry (MS) Unit
15... Mobile Phase Flow Path
151... Branch Point
152... Second Junction
16... Back Pressure Control Valve
171... Makeup Container
172... Makeup Flow Path
20... Sample
21... Agar Medium
211... Agar Medium Piece (A Part of Agar Medium)
22... Colonies of Microorganism
23... Pipette

## Claims

1. A method for analyzing a metabolite of a microorganism, the method comprising:
a step of supplying a mobile phase including carbon dioxide in a liquid state, in a subcritical state, or in a supercritical state to a container in which a microorganism cultured in a medium is contained together with the medium, to move a component of a metabolite of the microorganism contained in the microorganism and the medium to the mobile phase;
a step of introducing the mobile phase to which the component of the metabolite has moved to a column; and
a step of performing mass spectrometry on the component of the metabolite contained in the mobile phase that has passed through the column.

2. The method for quantifying a metabolite of a microorganism according to claim 1, the method comprising:
culturing the microorganism on an agar medium outside the container;
cutting the microorganism together with a part of the agar medium; and
containing the microorganism in the container together with a part of the agar medium.

3. The method for quantifying a metabolite of a microorganism according to claim 1, wherein
a component of a metabolite of the microorganism is an oil-soluble component, and
the mobile phase is a mixture of carbon dioxide and ammonium formate methanol.
